# EUROPEAN PATENT APPLICATION

(11) **EP 4 372 759 A1**
(43) Date of publication of application: **22.05.2024**
(21) Application number: 23790538.5
(22) Date of filing: 25.09.2023
(51) Int. Cl.: G16H 50/20, G16H 50/70, G01N 21/65, G01N 21/25, G06N 3/08, B82B 1/00, B82B 3/00

(54) **DEEP LEARNING AND SELF-ASSEMBLED-MONOLAYER-BASED ALZHEIMER'S DISEASE DIAGNOSIS METHOD, AND SERS SUBSTRATE THEREFOR**

(30) Priority: 26.09.2022 KR 20220121672
(71) Applicant: Korea Advanced Institute of Science and Technology, Daejeon 34141 (KR); KOREA INSTITUTE OF MATERIALS SCIENCE, Changwon-si, Gyeongsangnam-do 51508 (KR)
(72) Inventor: JUNG, YeonSik, Daejeon 34141 (KR); KIM, Minjoon, Daejeon 34141 (KR); HUH, Sejoon, Daejeon 34141 (KR); JO, Sungho, Daejeon 34141 (KR); PARK, Hyung Joon, Daejeon 34141 (KR); LEE, Min young, Changwon-si, Gyeongsangnam-do 51508 (KR)
(74) Representative: V.O.
(86) International application number: PCT/KR2023/014700
(87) International publication number: WO 2024/071926

(57) **Abstract**

Disclosed is a method for diagnosing Alzheimer's disease based on deep learning and an SAM, and a SERS substrate therefor. According to an embodiment, a deep learning and self-assembled monolayer (SAM)-based Alzheimer's disease diagnosing method performed by a computer device includes preparing a three-dimensional (3D) surface-enhanced Raman scattering (SERS) substrate by continuously stacking nanowire layers arranged in parallel by using a nanotransfer printing technology, forming an SAM on the 3D SERS substrate, and obtaining a Raman signal by applying a metabolite solution on the 3D SERS substrate having the SAM on a surface.

## Description

### [TECHNICAL FIELD]

Embodiments of the present disclosure described herein relate to a method for diagnosing Alzheimer's disease based on deep learning and a self-assembled monolayer (SAM), and a surface-enhanced Raman scattering (SERS) substrate therefor.

### [BACKGROUND ART]

SERS provides excellent sensitivity and speed through optical amplification using plasmonic nanostructures, as well as portability and non-destructiveness using a portable measurement device by measuring a Raman signal inherent in a molecule.

The SERS in biomedical diagnosis using the SERS has attempted a variety of methods including label-free detection of specific biomolecules and signal analysis of biomolecules mixed in cells or body fluids. The variety of methods may be mainly classified into targeted or untargeted label-free detection of major biomolecules such as proteins and DNA. As compared to conventional analysis methods such as ELISA and PCR, the label-free detection of the SERS has high sensitivity and specificity, resulting in fast detection speed and convenience.

Nowadays, there are two main methods of diagnosing Alzheimer's disease such as amyloid positron emission tomography (Amyloid-PET) and cerebrospinal fluid testing. These tests have high accuracy. However, the amyloid-PET is very expensive and the cerebrospinal fluid testing is invasive. Moreover, both the methods require patients to spend a lot of time in the clinic before being tested. Accordingly, there is a lot of research on a method of more easily diagnosing Alzheimer's disease by using blood. As part of the method, the potential of plasma proteins and metabolites is being studied as biomarkers.

It is (in recent years) theorized that Alzheimer's disease is developed when surplus reactive oxygen species are generated during metabolic processes in the mitochondria of brain cells, and the reactive oxygen species harm components of brain cells (by causing cell dysfunction or cell death). The metabolites refer to all the kinds of byproducts generated during these metabolic processes. Nowadays, many diseases are being linked to metabolic processes, and thus early diagnosis using the metabolites is being attempted.

Korean Patent Publication No. 10-2017-0032093 describes a nanotransfer printing method and SERS substrates, SERS vials, and SERS patches, which are fabricated by using the nanotransfer printing method.

### [DETAILED DESCRIPTION OF THE INVENTION]

### [TECHINICAL PROBLEM]

Embodiments of the present disclosure relate to a method for diagnosing Alzheimer's disease based on deep learning and SAM, and a SERS substrate therefor, and more particularly, provide a technology of performing label-free detection of plasma metabolites by using a three-dimensional (3D) SERS substrate with SAM formed, and classifying sample signals into sample signals of patients with Alzheimer's disease and sample signals of normal people by using deep learning.

Embodiments of the present disclosure provide a method for diagnosing Alzheimer's disease based on deep learning and an SAM, which classifies sample signals into sample signals of patients with Alzheimer's disease and sample signals of normal people by forming various SAMs on a 3D SERS substrate, applying plasma metabolites on the various SAMs, and measuring the amplified Raman signal (SERS signal), and a SERS substrate therefor.

### [TECHNICAL SOLUTION]

According to an embodiment, a deep learning and self-assembled monolayer (SAM)-based Alzheimer's disease diagnosing method performed by a computer device may include preparing a three-dimensional (3D) surface-enhanced Raman scattering (SERS) substrate by continuously stacking nanowire layers arranged in parallel by using a nanotransfer printing technology, forming an SAM on the 3D SERS substrate, and obtaining a Raman signal by applying a metabolite solution on the 3D SERS substrate having the SAM on a surface.

The method may further include diagnosing the Alzheimer's disease by classifying the obtained Raman signal through machine learning analysis.

The preparing of the 3D SERS substrate may include manufacturing an Au-based 3D SERS substrate by alternately stacking the nanowire layers arranged in parallel by using the nanotransfer printing technology.

The forming of the SAM on the 3D SERS substrate may include forming a plurality of different SAMs on the 3D SERS substrate.

According to another embodiment, a SERS substrate for diagnosing Alzheimer's disease based on deep learning and an SAM may include a 3D SERS substrate formed by continuously stacking nanowire layers arranged in parallel by using a nanotransfer printing technology, and an SAM formed on the 3D SERS substrate. The SERS substrate may obtain a Raman signal by applying a metabolite solution on the 3D SERS substrate having the SAM, and may classify the obtained Raman signal through machine learning analysis to diagnose Alzheimer's disease.

The 3D SERS substrate may be an Au-based 3D SERS substrate formed by alternately stacking the nanowire layers arranged in parallel by using the nano-transfer printing technology.

The SAM may be poly methyl methacrylate (PMMA) that remains on a surface of a transferred nanowire as a residue in a process of manufacturing the 3D SERS substrate.

The SAM may be a methyl group (-CH3) formed by bonding Au and a thiol group of 1-propanethiol.

The SAM may be a carboxylic acid group generated by performing O₂ plasma treatment on a surface of the 3D SERS substrate.

The SAM may be an amide group formed through amine coupling between a carboxylic acid film and ethylenediamine.

### [ADVANTAGEOUS EFFECTS OF THE INVENTION]

According to embodiments, a method for diagnosing Alzheimer's disease based on deep learning and a SAM, which classifies sample signals into sample signals of patients with Alzheimer's disease and sample signals of normal people by forming various SAMs on a 3D SERS substrate, applying plasma metabolites on the various SAMs, and measuring the amplified Raman signal (SERS signal), and a SERS substrate therefor are provided.

### [DESCRIPTION OF THE DRAWINGS]

FIG. 1 is a diagram for describing a deep learning and SAM-based Alzheimer's disease diagnosing method by using a SERS structure, according to an embodiment.
FIG. 2 is a diagram for describing a method of manufacturing a SERS substrate for deep learning and SAM-based Alzheimer's disease diagnosis, according to an embodiment.
FIGS. 3A and 3B are diagrams showing SEM images of a SERS substrate for deep learning and SAM-based Alzheimer's disease diagnosis, according to an embodiment.
FIG. 4 is a flowchart showing a deep learning and SAM-based Alzheimer's disease diagnosing method, according to an embodiment.
FIG. 5 is a diagram for describing localized surface plasmon resonance (LSPR) of a SERS substrate, according to an embodiment.
FIG. 6 is a diagram showing a TEM image of a SERS substrate, according to an embodiment.
FIG. 7 is a diagram for describing a process of a SAM-SERS substrate, according to an embodiment.
FIG. 8 is a diagram for describing a contact angle of a SAM-SERS substrate, according to an embodiment.
FIG. 9 is a diagram illustrating Raman spectra of a SAM-SERS substrate, according to an embodiment.
FIG. 10 is a diagram illustrating a structure of 5-Carboxytetramethylrhodamine (5-TAMRA), according to an embodiment.
FIGS. 11A to 11D are diagrams showing 5-TAMRA signals on a SAM-SERS substrate, according to an embodiment.
FIG. 12 is a diagram illustrating a structure of p-Phenylenediamine (p-PDA), according to an embodiment.
FIG. 13A to 13D are diagrams showing p-PDA signals on a SAM-SERS substrate, according to an embodiment.
FIG. 14 is a diagram illustrating a structure of a folic acid, according to an embodiment.
FIGS. 15A to 15D are diagrams illustrating folic acid signals on a SAM-SERS substrate, according to an embodiment.
FIG. 16 is a diagram for describing metabolite extraction and Raman signal measurement, according to an embodiment.
FIGS. 17A to 17C are drawings for describing results of analyzing metabolite solution signals, according to an embodiment.
FIGS. 18A to 18D are drawings for describing results of analyzing metabolite solution signals for distinguishing between AD and HC for each SAM coating type, according to an embodiment.

### [BEST MODE]

Hereinafter, embodiments of the present disclosure will be described with reference to accompanying drawings. However, embodiments to be described may be modified in the different forms, and the scope and spirit of the present disclosure is not limited by the embodiments to be described below. In addition, various embodiments are provided to describe the present disclosure more fully to those skilled in the art. For a clear description, forms, sizes, and the like of elements may be exaggerated in a drawing.

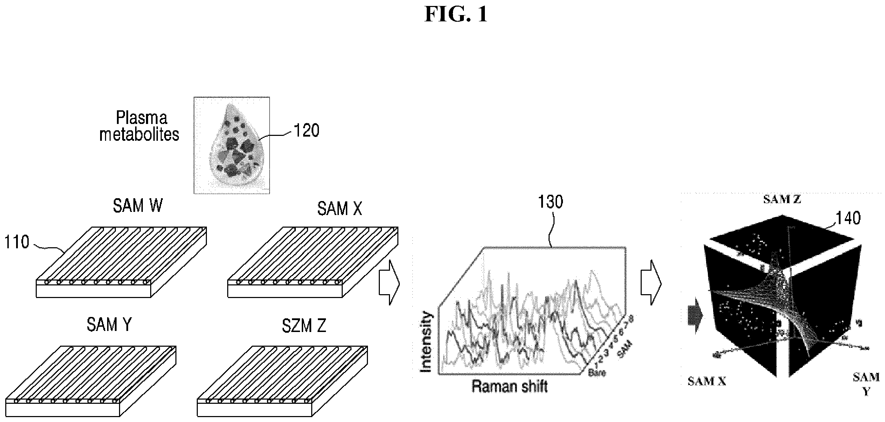

FIG. 1 is a diagram for describing a deep learning and SAM-based Alzheimer's disease diagnosing method by using a SERS structure, according to an embodiment.

Metabolites may be generally small molecules, and thus may cross a blood-brain barrier. For example, small molecules under 1,500 Da, 150,000 metabolites or more, and metabolites related to Alzheimer's disease may cross the blood-brain barrier. Here, the metabolites related to Alzheimer's disease include Glycerate, Serine, N-Acetylneuraminate, Sphingolipid, and 2-Hydroxybutyrate.

According to an embodiment, a deep learning and SAM-based Alzheimer's disease diagnosing method using an SERS structure may obtain a variety of Raman spectra. Moreover, high-dimensional deep learning analysis is possible to identify even subtle differences.

Referring to FIG. 1, the deep learning and SAM-based Alzheimer's disease diagnosis method using the SERS structure according to an embodiment may measure (130) a Raman signal (SERS signal) amplified by forming various SAMs (e.g., SAM W, SAM X, SAM Y, SAM Z 110) on a 3D SERS substrate, and applying plasma metabolites 120 on the SAMs.

When an SAM of a substrate is different even though the analyte is the same, a difference in dipole interaction between the analyte and the SAM may occur. This difference changes in spacing and directionality between the analyte and the localized surface plasmon resonance (LSPR) to generate various Raman signals. In performing machine learning 140, this diversity may ultimately allow multi-dimensional analysis to be performed, thereby improving the accuracy of signal classification.

In this way, label-free detection of plasma metabolites may be performed by using the 3D SERS structure formed with the SAM, and sample signals may be classified into sample signals of patients with Alzheimer's disease and sample signals of normal people by using deep learning.

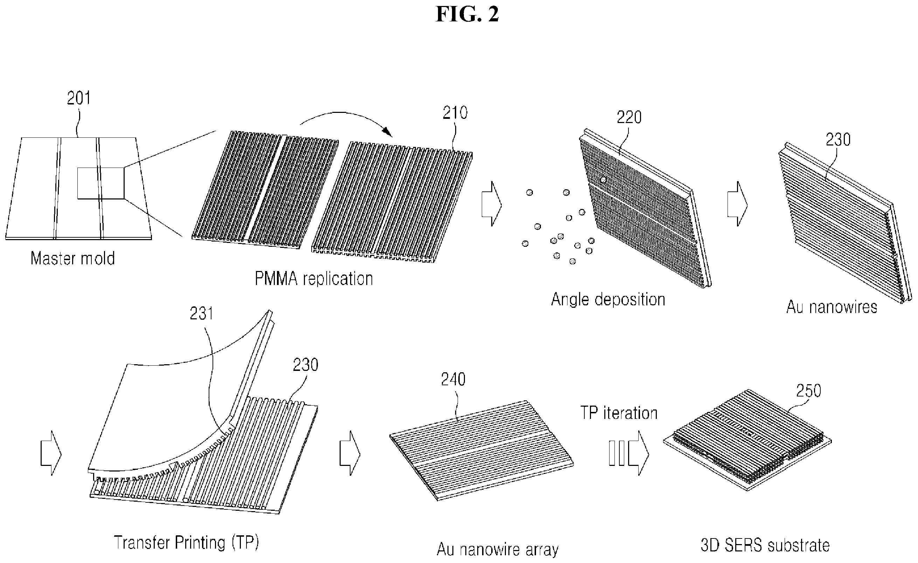

FIG. 2 is a diagram for describing a method of manufacturing a SERS substrate for deep learning and SAM-based Alzheimer's disease diagnosis, according to an embodiment.

Referring to FIG. 2, a gold (Au)-based 3D plasmonic SERS structure substrate may be manufactured by successively stacking (alternately stacking) nanowire layers arranged in parallel by using a nanotransfer printing technology (nTP).

Poly methyl methacrylate (PMMA) replication 210 may be performed by selecting at least part of a master mold 201. Au nanowires 230 may be formed on the replicated PMMA substrate 210 through angle deposition 220. Moreover, an Au nanowire array 240 may be formed through transfer printing (TP) 231, and a 3D SERS substrate 250 may be formed through transfer printing iteration.

For example, the SERS substrate may be manufactured by a method including: coating a polymer thin film on a template substrate with a surface pattern formed, manufacturing the polymer thin film into a replica thin film mold by using the polymer thin film and an adhesive film, forming a nanostructure on the replica thin film mold, selectively weakening adhesive force between the adhesive film and the replica thin film mold, and transferring the nanostructure to a target object.

In more detail, a first process may include coating a polymer thin film on a template substrate with a surface pattern formed, manufacturing the polymer thin film into a replica thin film mold by using the polymer thin film and an adhesive film, and forming a nanostructure on the replica thin film mold. In this case, an uneven surface pattern may be formed on the template substrate by using a patterning process including at least one of photolithography, block copolymer self-assembly-based lithography, or Ebeam lithography, and a reactive ion etching (RIE) process.

Moreover, the polymer thin film may be coated by applying the polymer thin film on the template substrate by using at least one process of spin coating, deep coating, or spray coating. In particular, the nanostructure may be formed by depositing a functional material on the replica thin film mold in a method of the angle deposition.

When this first process is completed, a second process is performed to selectively weaken the adhesive force between the adhesive film and the replica thin film mold, and then to transfer the nanostructure to the target object. In this case, the adhesive force between the adhesive film and the replica thin film mold may be selectively weakened by injecting organic solvent vapor between the adhesive film and the replica thin film mold to reduce the separation energy between the interfaces.

In this way, a SERS substrate used to analyze components of a material may be manufactured by forming a nanostructure of a metal material such as Au and performing nanotransfer printing on the target object. In the meantime, the SERS substrate may include the nanotransfer printing method described in Korean Patent Publication No. 10-2017-0032093 and a SERS substrate, SERS vial, and SERS patch, which are manufactured using the same.

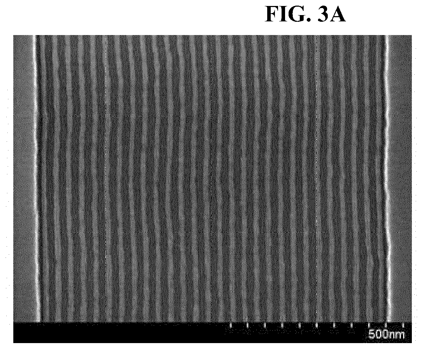

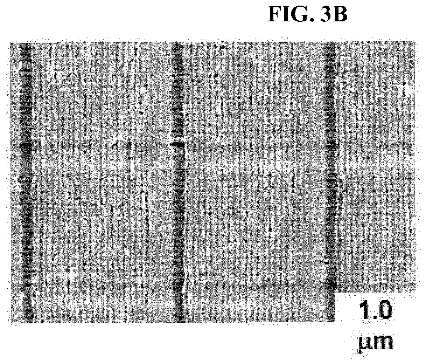

FIGS. 3A and 3B are diagrams showing SEM images of a SERS substrate for deep learning and SAM-based Alzheimer's disease diagnosis, according to an embodiment.

Referring to FIG. 3A, it shows an SEM image of a master mold according to an embodiment. Referring to FIG. 3B, it shows an SEM image of a 3D SERS substrate according to an embodiment.

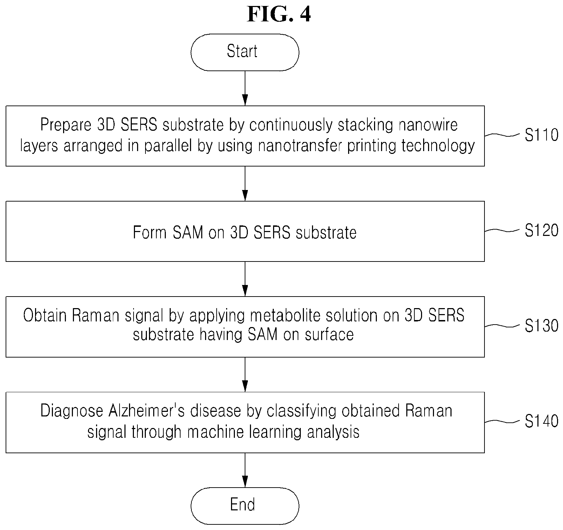

FIG. 4 is a flowchart showing a deep learning and SAM-based Alzheimer's disease diagnosing method, according to an embodiment.

Referring to FIG. 4, a deep learning and SAM-based Alzheimer's disease diagnosing method performed by a computer device according to an embodiment may include preparing a 3D SERS substrate by continuously stacking nanowire layers arranged in parallel by using a nanotransfer printing technology (S110), forming an SAM on a 3D SERS substrate (S120), and obtaining a Raman signal by applying a metabolite solution on the 3D SERS substrate having the SAM on a surface (S130).

In addition, the Alzheimer's disease diagnosing method may further include diagnosing Alzheimer's disease by classifying the obtained Raman signal through machine learning analysis (S140).

Hereinafter, the deep learning and SAM-based Alzheimer's disease diagnosing method according to an embodiment will be described in more detail.

In operation S110, the Alzheimer's disease diagnosing method may include preparing a 3D SERS substrate by continuously stacking nanowire layers arranged in parallel by using a nanotransfer printing technology. In this case, an Au-based 3D SERS substrate may be manufactured by alternately stacking nanowire layers arranged in parallel by using a nanotransfer printing technology.

In operation S120, an SAM may be formed on the 3D SERS substrate. Here, a plurality of different SAMs may be formed on the 3D SERS substrate.

The SAM may be PMMA that remains on a surface of the transferred nanowire as a residue in a process of manufacturing the 3D SERS substrate.

Moreover, the SAM may be a methyl group (-CH3) formed by bonding Au and the thiol group of 1-propanethiol.

Furthermore, the SAM may be a carboxylic acid group generated by performing O₂ plasma treatment on a surface of the 3D SERS substrate.

Besides, the SAM may be an amide group formed through amine coupling between a carboxylic acid film and ethylenediamine.

In operation S130, the Raman signal may be obtained by applying the metabolite solution on the 3D SERS substrate having the SAM on a surface.

According to an embodiment, the deep learning and SAM-based Alzheimer's disease diagnosing method using a SERS substrate includes extracting metabolites from plasma components. For example, plasma of 100 µL and a solution from mixing methanol and ethanol at 8:2 may be mixed and centrifuged to extract the supernatant.

In operation S140, Alzheimer's disease may be diagnosed by classifying the obtained Raman signal through machine learning analysis.

According to an embodiment, the deep learning and SAM-based Alzheimer's disease diagnosing method using a SERS substrate may obtain a Raman signal. In this case, the metabolite solution may be dropped on the 3D SERS substrate having various functional groups on the surface, and then a Raman signal may be obtained by using an excitation laser of 785 nm. Moreover, Alzheimer's disease may be diagnosed by classifying the obtained Raman signal through machine learning analysis.

As such, according to embodiments, blood is used unlike a conventional diagnosing method, and Alzheimer's disease may be early diagnosed by using Raman. In particular, according to embodiments, the accuracy may be increased by utilizing machine learning.

According to an embodiment, the SERS substrate for deep learning and SAM-based Alzheimer's disease diagnosis may include a 3D SERS substrate formed by continuously stacking nanowire layers arranged in parallel by using a nanotransfer printing technology, and an SAM formed on the 3D SERS substrate. The SERS substrate may obtain a Raman signal by applying a metabolite solution on the 3D SERS substrate having the SAM, and may classify the obtained Raman signal through machine learning analysis to diagnose Alzheimer's disease.

Here, a 3D SERS substrate may be an Au-based 3D SERS substrate manufactured by alternately stacking nanowire layers arranged in parallel by using the nanotransfer printing technology.

Furthermore, the SAM may form a plurality of different SAMs on the 3D SERS substrate.

According to embodiments, label-free detection of plasma metabolites may be performed by using the 3D SERS substrate formed with the SAM, and sample signals may be classified into sample signals of patients with Alzheimer's disease and sample signals of normal people by using deep learning.

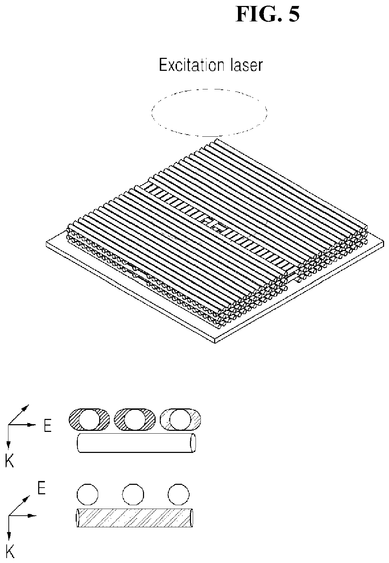

FIG. 5 is a diagram for describing localized surface plasmon resonance (LSPR) of a SERS substrate, according to an embodiment.

As shown in FIG. 5, on a 3D SERS substrate, which is formed by alternately stacking nanowire layers, LSPR is formed around nanowires regardless of the polarization direction of incident light. Moreover, the 3D SERS substrate may further amplify a Raman signal of an analyte as a gap between horizontal and vertical directions creates a hot spot that is an area where LSPRs overlap each other.

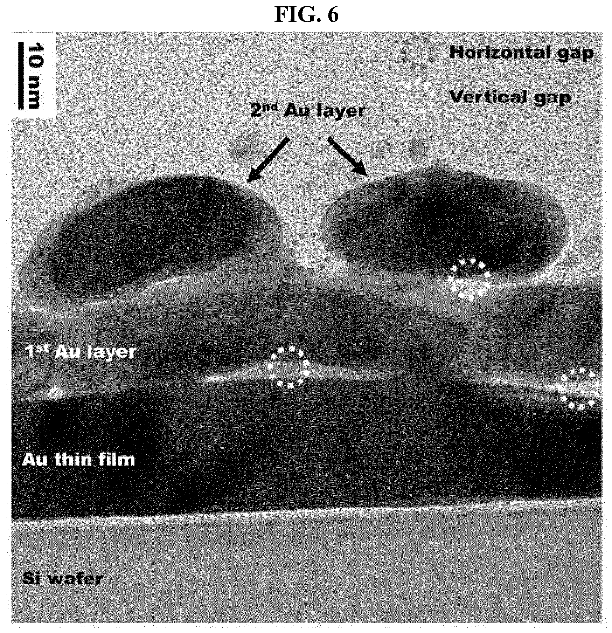

FIG. 6 is a diagram showing a TEM image of a SERS substrate, according to an embodiment.

As shown in FIG. 6, an Au thin film may be formed on a Si wafer, and a first Au layer and a second Au layer may be formed on the Au thin film. The 3D SERS substrate formed by alternately stacking nanowire layers may further amplify a Raman signal of an analyte as horizontal and vertical gaps creates a hot spot that is an area where LSPRs overlap each other. For example, a horizontal gap may be formed between the second Au layers. The vertical gap may be formed between the Au thin film and the first Au layer or between the first Au layer and the second Au layer.

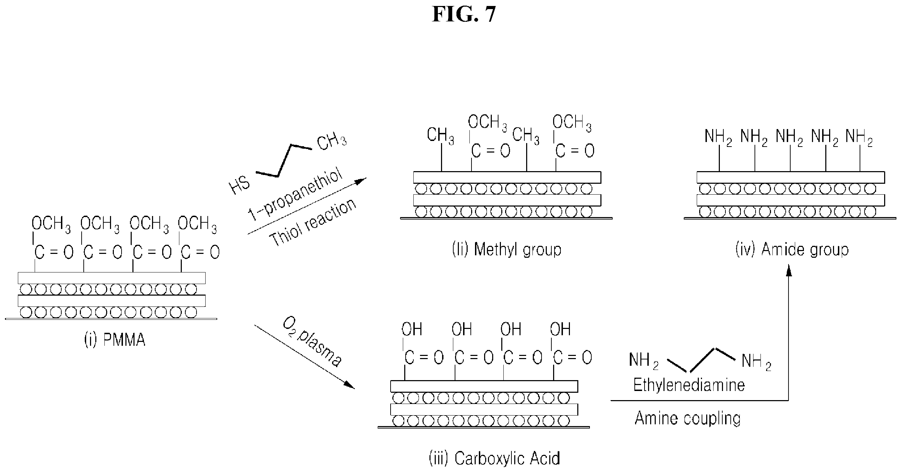

FIG. 7 is a diagram for describing a process of a SAM-SERS substrate, according to an embodiment.

Referring to FIG. 7, a total of four different SAM layers are formed on a 3D SERS substrate through a process of a SAM-SERS substrate according to an embodiment. Here, the four SAM-SERS substrates include a PMMA, a methyl group, a carboxylic acid, and an amide group.

PMMA: the PMMA remains on a surface of a transferred nanowire as a residue in a process. In other words, the PMMA may utilize a native PMMA layer generated on a surface when a SERS substrate is manufactured without special treatment.

Methyl group: a methyl group (-CH3) may be formed by bonding Au and a thiol group of 1-propanethiol. In other words, the methyl group may be attached by reacting a thiol group at one end of 1 -propanethiol with the surface of the SERS substrate.

Carboxylic acid: a carboxylic acid surface is generated by performing O₂ plasma treatment on a PMMA film (using reactive ion etching equipment). In other words, the carboxylic acid may generate a carboxylic acid group by performing O₂ plasma treatment on the surface of the SERS substrate.

Amide group: an amide group is formed through amine coupling between a carboxylic acid film and ethylenediamine. In other words, the amide group may attach ethylenediamine to the SERS substrate, on which the carboxylic acid group is formed, through an amine coupling reaction.

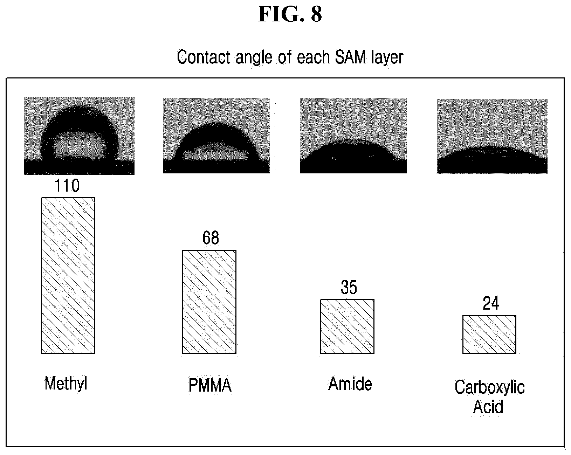

FIG. 8 is a diagram for describing a contact angle of a SAM-SERS substrate, according to an embodiment.

Referring to FIG. 8, the contact angle of a SERS substrate, on which a SAM layer is formed, has been measured differently for each type (24 to 110). For example, a methyl group is 110; a PMMA is 68; an amide group is 35; and a carboxylic acid is 24.

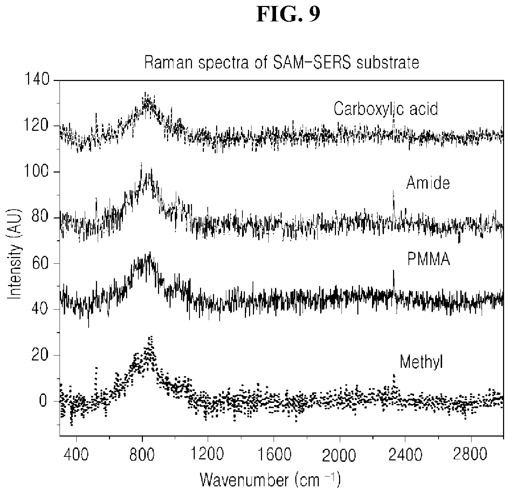

FIG. 9 is a diagram illustrating Raman spectra of a SAM-SERS substrate, according to an embodiment.

Referring to FIG. 9, it may be seen that a signal of a SAM is not significantly observed when a Raman signal of a SAM layer on a SERS substrate is measured, and thus there is no need to worry about signal interference with an analyte.

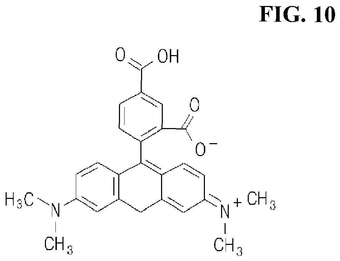

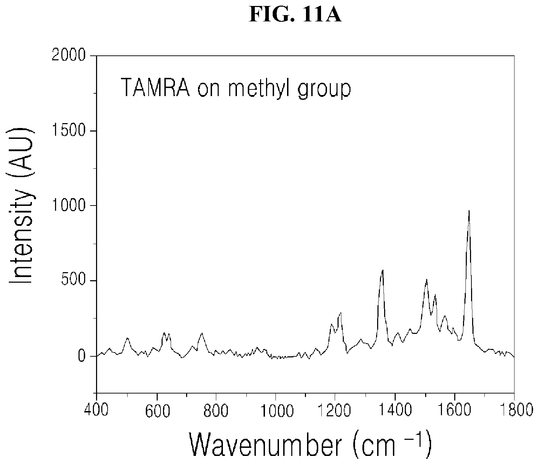

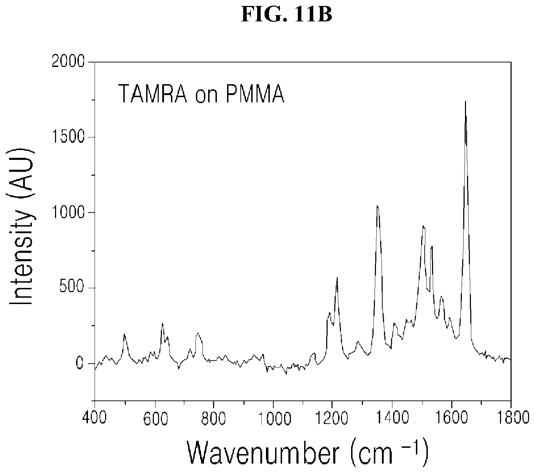

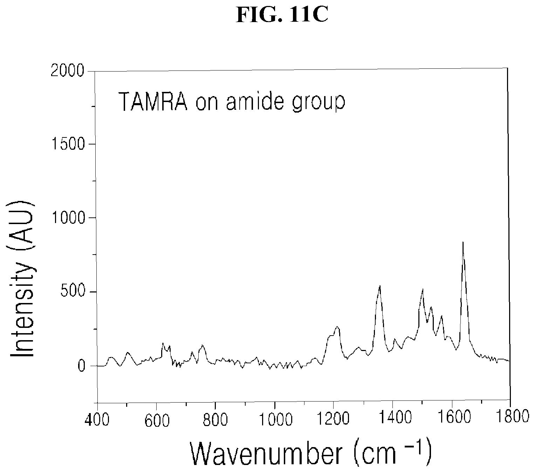

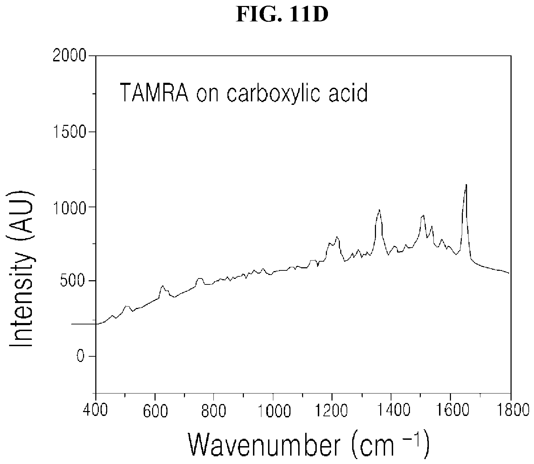

FIG. 10 is a diagram illustrating a structure of 5-Carboxytetramethylrhodamine (5-TAMRA), according to an embodiment. FIGS. 11A to 11D are diagrams showing 5-TAMRA signals on a SAM-SERS substrate, according to an embodiment.

Referring to FIGS. 10 and 11A to 11D, the 5-TAMRA signal may be identified on the SAM-SERS substrate. Specific analytes have different dipole interactions depending on the type of a SAM layer. As a result, different orientations and gaps between the analyte and the 3D SERS substrate cause changes in Raman signals. The changes in Raman signals according to the SAM layer with a single substance are observed before several metabolites are complexly analyzed.

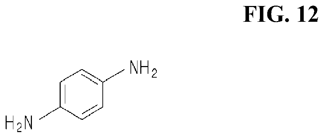

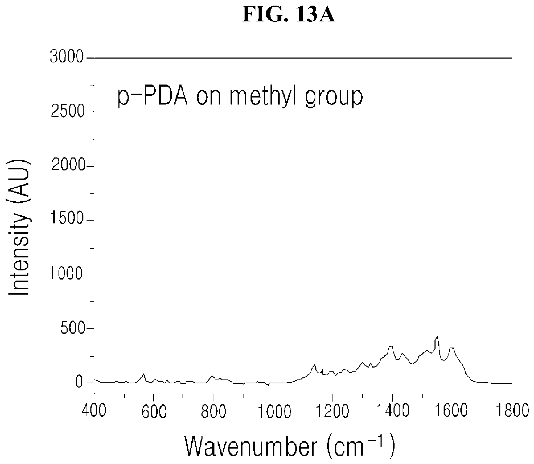

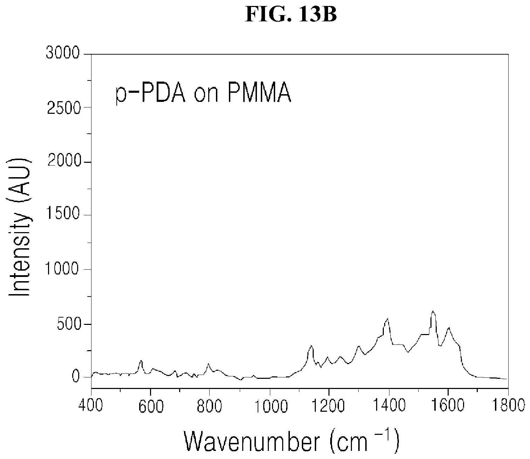

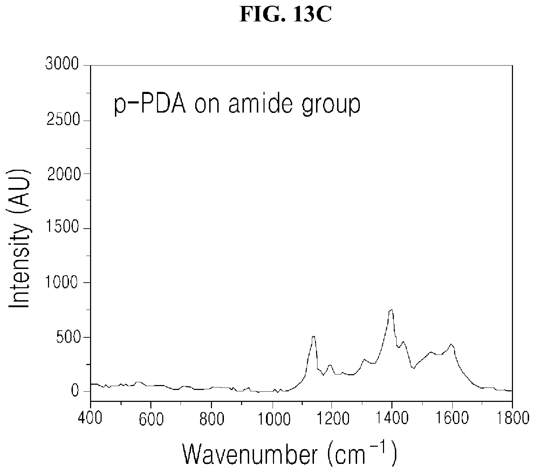

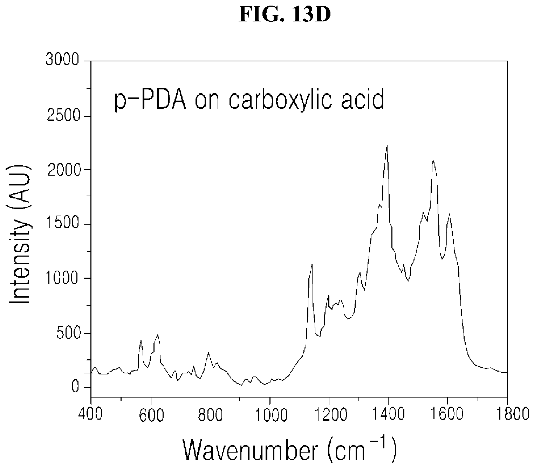

FIG. 12 is a diagram illustrating a structure of p-Phenylenediamine (p-PDA), according to an embodiment. FIG. 13A to 13D are diagrams showing p-PDA signals on a SAM-SERS substrate, according to an embodiment.

Referring to FIGS. 12 and 13A to 13D, the p-PDA signals on the SAM-SERS substrate may be identified. The p-PDA has a relatively simple molecular structure, but has large changes in Raman signals for each SAM coating type.

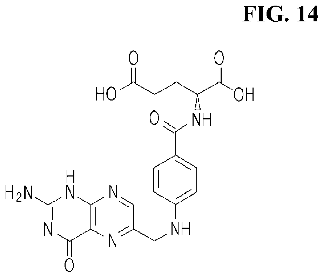

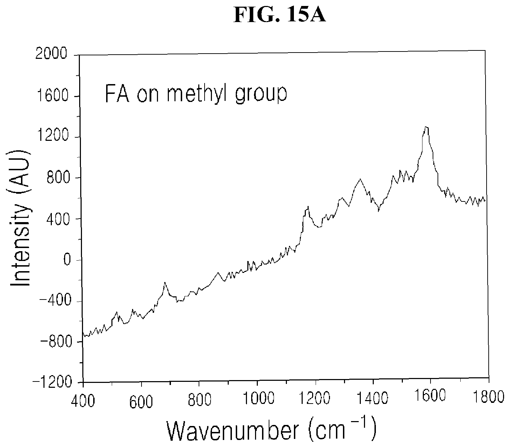

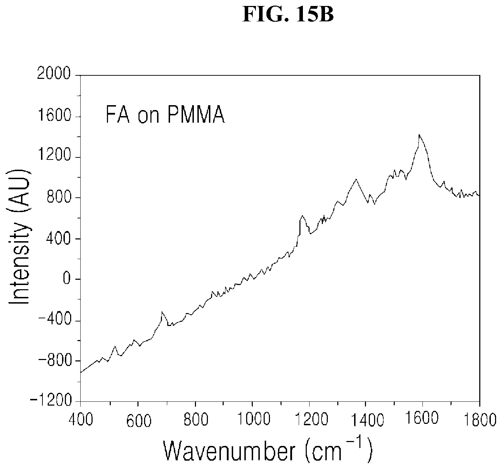

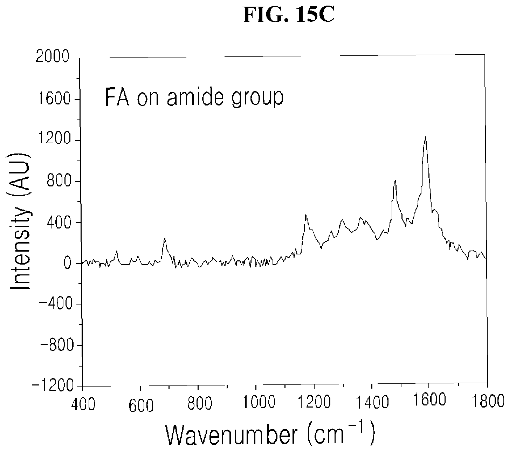

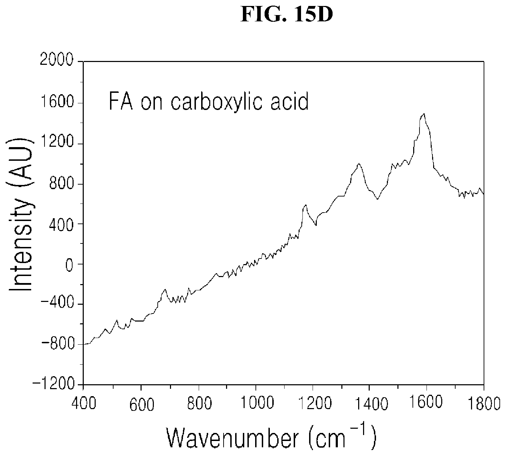

FIG. 14 is a diagram illustrating a structure of a folic acid, according to an embodiment. FIGS. 15A to 15D are diagrams illustrating folic acid signals on a SAM-SERS substrate, according to an embodiment.

Referring to FIGS. 14 and 15A to 15D, the folic acid signals on the SAM-SERS substrate may be identified. The folic acid is a single substance having a relatively large Raman cross section. Different types of SERS signals are generated for each type of a SAM. In particular, it may be seen that a fluorescence background signal is reduced at an amide group.

As such, it may be seen that Raman signals, of which types are slightly different from each other, are generated on different SAM-SERS substrates by using a single substance having a relatively large Raman cross section, such as a folic acid.

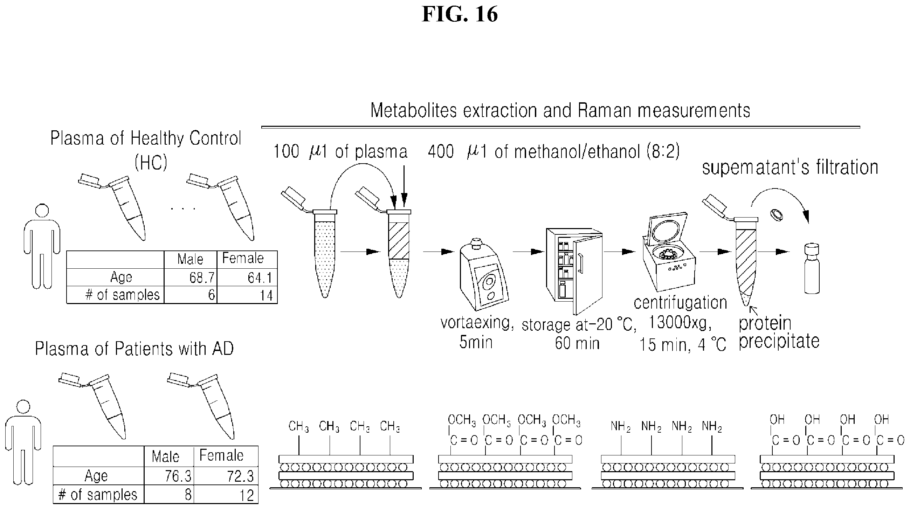

FIG. 16 is a diagram for describing metabolite extraction and Raman signal measurement, according to an embodiment.

Referring to FIG. 16, experiments of metabolite extraction and Raman signal measurement according to an embodiment may be performed. Here, plasma samples have been obtained from 20 healthy controls (HC) and 20 patients with Alzheimer's disease (AD). When being mixed in a methanol/ethanol mixture, plasma samples are separated by using a centrifuge into a protein precipitate and a supernatant solution (including metabolites). After metabolite solutions are dropped onto four SAM-SERS substrates, Raman measurements are performed 20 times per sample. Here, the four SAM-SERS substrates include a PMMA, a methyl group, a carboxylic acid, and an amide group.

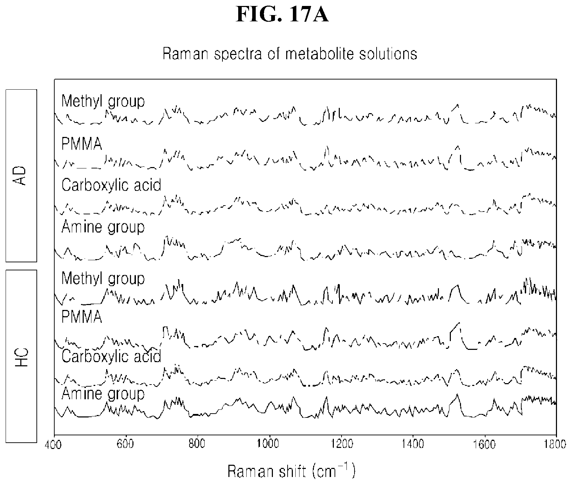

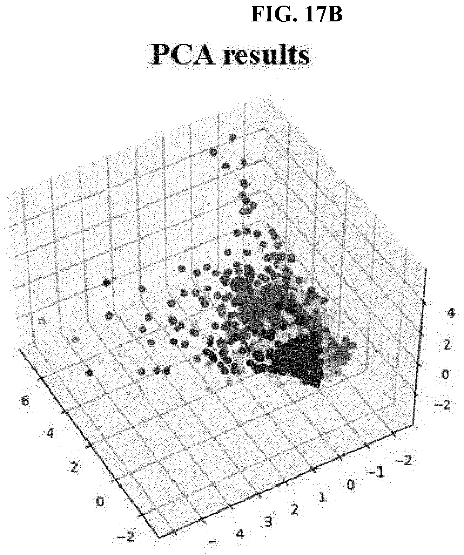

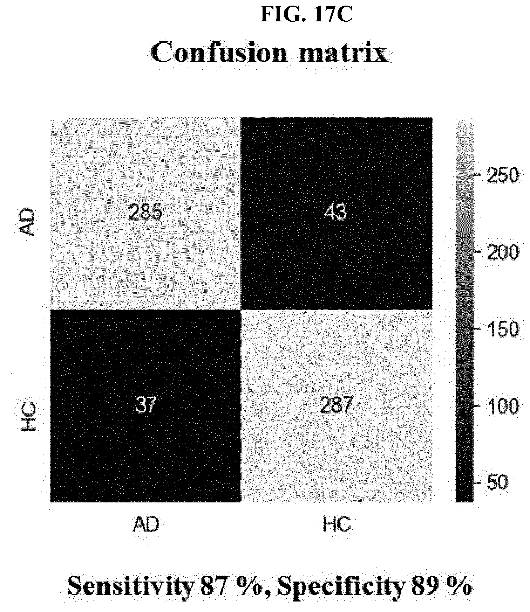

FIGS. 17A to 17C are drawings for describing results of analyzing metabolite solution signals, according to an embodiment.

Referring to FIGS. 17A to 17C, results of analyzing metabolite solution signals according to an embodiment indicate that Raman signals of a healthy control (HC) and Raman signals of patients with Alzheimer's disease (AD) are indistinguishable from each other with naked eyes. Furthermore, referring to a principal component analysis (PCA) spatial graph, there are no obvious clusters of HC or AC points, and thus the classification through PCA analysis is also difficult.

Here, sensitivity of 87% and specificity of 89% have been obtained when HC and AC signals are classified by using deep learning regardless of a SAM type. In this way, it may be seen that the analysis performance is clearly improved when deep learning is used.

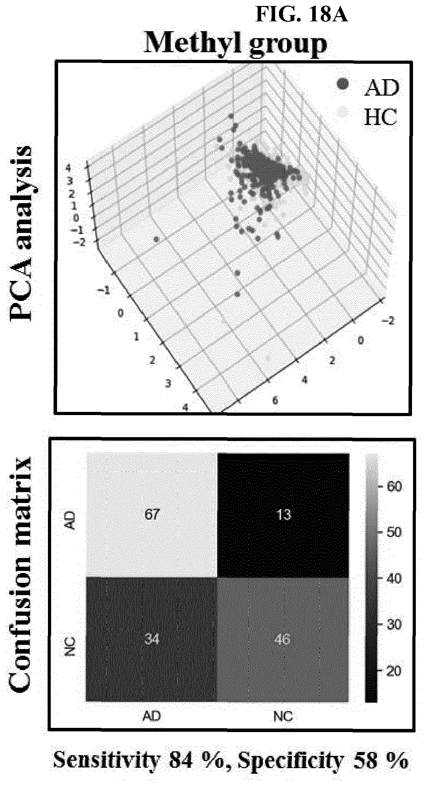

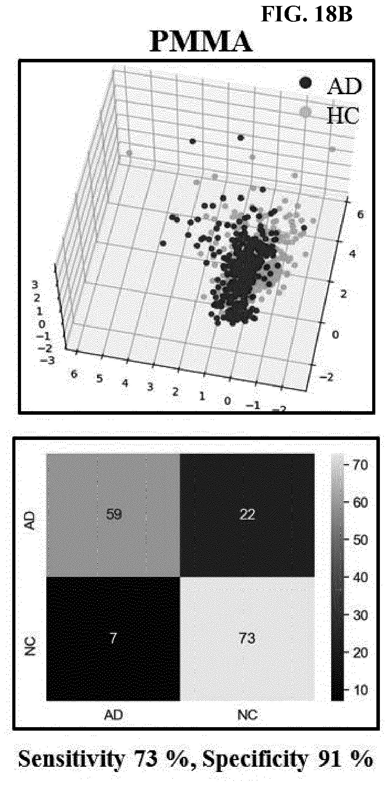

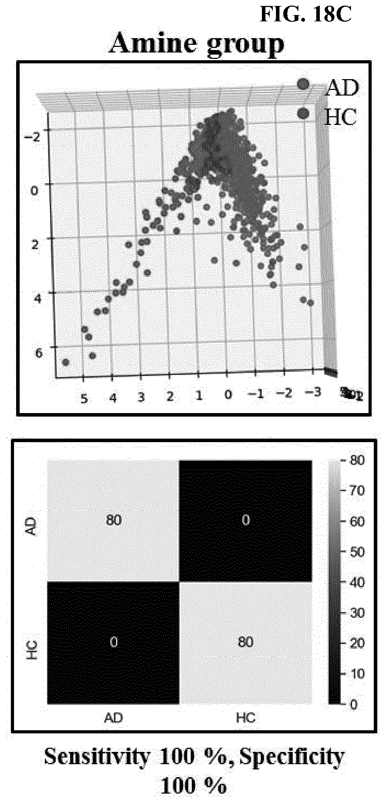

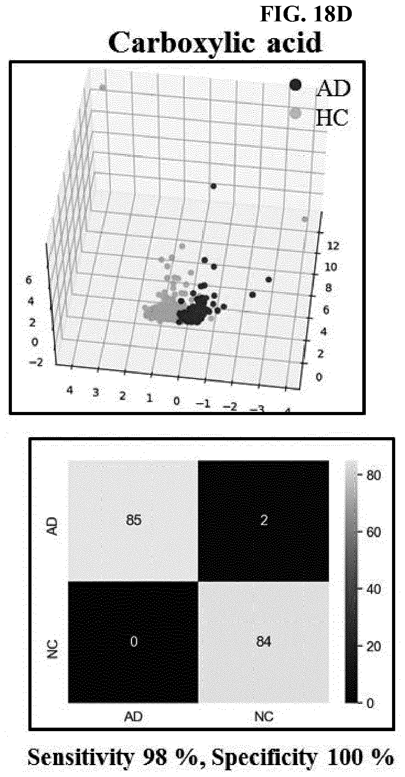

FIGS. 18A to 18D are drawings for describing results of analyzing metabolite solution signals for distinguishing between AD and HC for each SAM coating type, according to an embodiment.

Referring to FIGS. 18A to 18D, results of distinguishing between AD and HC for each SAM coating type of a SERS substrate according to an embodiment are shown. A substrate, which is a substrate having higher hydrophilicity (having a lower contact angle) and which is coated with an amide group and a carboxylic acid, may have higher performance. The penetration of metabolite solutions in a depth direction or horizontal spreading of the metabolite solutions due to high surface energy of the metabolite solutions may be an advantage in mixture analysis.

As described above, according to embodiments, sample signals may be classified into sample signals of patients with Alzheimer's disease and sample signals of normal people by forming various SAMs on a 3D SERS substrate, applying plasma metabolites on the various SAMs, and measuring the amplified Raman signal (SERS signal). In particular, according to embodiments, Raman signals of healthy controls (HCs) and patients with Alzheimer's disease (AD), which are difficult to distinguish between each other with naked eyes, may be distinguished between each other by using deep learning, thereby making it possible to be analyzed distinctly.

In this way, Alzheimer's disease may be early diagnosed by using SERS and machine learning.

The foregoing devices may be realized by hardware elements, software elements and/or combinations thereof. For example, the devices and components described in the exemplary embodiments of the present disclosure may be implemented in one or more general-use computers or special-purpose computers, such as a processor, a controller, an arithmetic logic unit (ALU), a digital signal processor, a microcomputer, a field programmable array (FPA), a programmable logic unit (PLU), a microprocessor or any device which may execute instructions and respond. A processing unit may perform an operating system (OS) or one or software applications running on the OS. Further, the processing unit may access, store, manipulate, process and generate data in response to execution of software. It will be understood by those skilled in the art that although a single processing unit may be illustrated for convenience of understanding, the processing unit may include a plurality of processing elements and/or a plurality of types of processing elements. For example, the processing unit may include a plurality of processors or one processor and one controller. Also, the processing unit may have a different processing configuration, such as a parallel processor.

Software may include computer programs, codes, instructions or one or more combinations thereof and configure a processing unit to operate in a desired manner or independently or collectively control the processing unit. Software and/or data may be embodied in any type of machine, components, physical equipment, virtual equipment, computer storage media or devices so as to be interpreted by the processing unit or to provide instructions or data to the processing unit. Software may be dispersed throughout computer systems connected via networks and be stored or executed in a dispersion manner. Software and data may be recorded in one or more computer-readable storage media.

The methods according to the above-described embodiments may be recorded in a computer-readable medium including program instructions that are executable through various computer devices. The computer-readable medium may also include the program instructions, data files, data structures, or a combination thereof. The program instructions recorded in the media may be designed and configured especially for the example embodiments or be known and available to those skilled in computer software. The computer-readable medium may include hardware devices, which are specially configured to store and execute program instructions, such as magnetic media (e.g., a hard disk, a floppy disk, or a magnetic tape), optical recording media (e.g., CD-ROM and DVD), magneto-optical media (e.g., a floptical disk), read only memories (ROMs), random access memories (RAMs), and flash memories. Examples of computer programs include not only machine language codes created by a compiler, but also high-level language codes that are capable of being executed by a computer by using an interpreter or the like.

While embodiments have been shown and described with reference to the accompanying drawings, it will be apparent to those skilled in the art that various modifications and variations may be made from the foregoing descriptions. For example, adequate effects may be achieved even if the foregoing processes and methods are carried out in different order than described above, and/or the aforementioned elements, such as systems, structures, devices, or circuits, are combined or coupled in different forms and modes than as described above or be substituted or switched with other components or equivalents.

Therefore, other implements, other embodiments, and equivalents to claims are within the scope of the following claims.

## Claims

1. A deep learning and self-assembled monolayer (SAM)-based Alzheimer's disease diagnosing method performed by a computer device, the method comprising:
preparing a three-dimensional (3D) surface-enhanced Raman scattering (SERS) substrate by continuously stacking nanowire layers arranged in parallel by using a nanotransfer printing technology;
forming an SAM on the 3D SERS substrate; and
obtaining a Raman signal by applying a metabolite solution on the 3D SERS substrate having the SAM on a surface.

2. The method of claim 1, further comprising:
diagnosing the Alzheimer's disease by classifying the obtained Raman signal through machine learning analysis.

3. The method of claim 1, wherein the preparing of the 3D SERS substrate includes:
manufacturing an Au-based 3D SERS substrate by alternately stacking the nanowire layers arranged in parallel by using the nanotransfer printing technology.

4. The method of claim 1, wherein the forming of the SAM on the 3D SERS substrate includes:
forming a plurality of different SAMs on the 3D SERS substrate.

5. A SERS substrate for diagnosing Alzheimer's disease based on deep learning and an SAM, the SERS substrate comprising:
a 3D SERS substrate formed by continuously stacking nanowire layers arranged in parallel by using a nanotransfer printing technology; and
an SAM formed on the 3D SERS substrate,
wherein the SERS substrate obtains a Raman signal by applying a metabolite solution on the 3D SERS substrate having the SAM, and classifies the obtained Raman signal through machine learning analysis to diagnose Alzheimer's disease.

6. The SERS substrate of claim 5, wherein the 3D SERS substrate is an Au-based 3D SERS substrate formed by alternately stacking the nanowire layers arranged in parallel by using the nano-transfer printing technology.

7. The SERS substrate of claim 5, wherein the SAM is poly methyl methacrylate (PMMA) that remains on a surface of a transferred nanowire as a residue in a process of manufacturing the 3D SERS substrate.

8. The SERS substrate of claim 5, wherein the SAM is a methyl group (-CH3) formed by bonding Au and a thiol group of 1-propanethiol.

9. The SERS substrate of claim 5, wherein the SAM is a carboxylic acid group generated by performing O₂ plasma treatment on a surface of the 3D SERS substrate.

10. The SERS substrate of claim 5, wherein the SAM is an amide group formed through amine coupling between a carboxylic acid film and ethylenediam ine.
